# NOUVEAU FASCICULE DE BREVET EUROPEEN

(11) **EP 0 734 728 B2**
(45) Date de publication et mention de la décision concernant l'opposition: **13.02.2008**
(45) Mention de la délivrance du brevet: 06.06.2001
(21) Numéro de dépôt: 96400456.8
(22) Date de dépôt: 04.03.1996
(51) Int. Cl.: A61K 38/22, A61K 39/395, A61Q 19/00

(54) **Utilisation d'un antagoniste de CGRP pour traiter les lichens et les prurits et composition obtenue**
Verwendung eines CGRP Antagonisten zur Behandlung von Flechten und Pruritus und so erhaltene Zubereitung
Use of a CGRP antagonist for the treatment of lichens and pruritis and composition so obtained

(30) Priorité: 28.03.1995 FR 9503627
(43) Date de publication de la demande: 02.10.1996
(73) Titulaire: L'ORÉAL, 75008 Paris (FR)
(72) Inventeur: De Lacharriere, Olivier, F-75015 Paris (FR); Breton, Lionel, F-78000 Versailles (FR)
(74) Mandataire: Bernstein, Claire Jacqueline

(56) Documents cités:
- WO-A-93/21911
- NEUROSCIENCE, vol. 48, no. 4, Juin 1992, pages 963-968, XP000577183 T.L. BUCKLEY ET AL.: "THE PARTIAL INHIBITION OF INFLAMMATORY RESPONSES INDUCED BY A CAPSAICIN USING THE FAB FRAGMENT OF A SELECTIVE CALCITONIN GENE-RELATED PEPTIDE ANTISERUM IN RABBIT SKIN"
- BRITISH JOURNAL OF PHARMACOLOGY, vol. 110, no. 2, 1993, pages 772-776, XP000563605 K. JANE ESCOTT ET AL.: "EFFECT OF CALCITONIN GENE-RELATED PEPTIDE ANTAGONIST (CGRP 8-37) ON SKIN VASODILATATION AND OEDEMA INDUCED BY STIMULATION OF THE RAT SAPHENOUS NERVE"
- BRITISH JOURNAL OF PHARMACOLOGY, vol. 104, no. 3, Novembre 1991, pages 738-742, XP000563606 S.R. HUGHES ET AL.: "A CALCITONIN GENE-RELATED PEPTIDE (CGRP) ANTAGONIST (CGRP 8-37) INHIBITS MICROVASCULAR REPONSES INDUCED BY CGRP AND CAPSAICIN IN SKIN."
- NEUROSCIENCE LETTERS, vol. 102, no. 2.3, 31 Juillet 1989, pages 257-260, XP000578094 S.M. LOUIS ET AL.: "ANTIBODIES TO CALCITONIN-GENE RELATED PEPTIDE REDUCE INFLAMMATION INDUCED BY TOPICAL MUSTARD OIL BUT NOT THAT DUE TO CARRAGEENIN IN THE RAT"
- Neuropeptides et Neuromédiateurs. J. EPELBAUM. Ed. Inserm 1995, pp71-78 et 281-294.
- Hayes N.A. et al, AGENTS AND ACTIONS, Vol. 38, Special Issue II, 1993, p.c212-c214
- EUROPEAN JOURNAL OF DERMATOLOGY, 1994,2(4), 154-58
- C.M: Maggi et al., EUROPEAN JOURNAL OF PHARMACOLOGY, VOL. 192, 1991, p. 85-88

## Description

La présente invention se rapporte à l'utilisation d'un antagoniste de CGRP (peptide dérivé du gène de la calcitonine : Calcitonin Gene Related Peptide en langue anglosaxonne) dans ou pour la préparation d'une composition cosmétique, pharmaceutique ou dermatologique, notamment à application topique, pour traiter certaines maladies de la peau.

Plus spécialement, cette composition permet le traitement par voie topique, ingérable ou injectable des lichens notamment les lichens plans ; des prurigos et en particulier les prurigos actinique, de Besnier ou Hebra ou strophulus ; et des toxidermies prurigineuses. Les toxidermies prurigineuses sont notamment consécutives à l'absorption de médicament ; ces maladies sont très différentes de l'urticaire et ne font intervenir aucune réaction de contact.

Jusqu'à ce jour, les lichens plans et pigmentaires étaient traités à l'aide de corticoïdes locaux ou de puvathérapie. Les corticoïdes sont certes très efficaces pour calmer les symptômes mais malheureusement, ils présentent des effets secondaires souvent très pénalisants comme des atrophies, des infections surtout mycosiques ou bactériennes. La puvathérapie est l'irradiation locale de la peau malade avec des UVA, après absorption d'une substance photosensibilisante (psoralène). Cette technique présente les inconvénients graves d'un photovieillissement pouvant entraîner le plus souvent des cancers de la peau. De plus, ce traitement n'est pas ambulatoire, obligeant les malades à se rendre couramment dans un centre spécialisé pendant toute la durée du traitement, ce qui est très contraignant et limite leur activité professionnelle.

Les prurigos sont aussi traités par les corticoïdes locaux, puvathérapie ou thalidomide. Les corticoïdes locaux et la puvathérapie présentent les inconvénients ci-dessus. La thalidomide présente l'inconvénient majeur d'être tératogène, ce qui interdit son utilisation chez la femme enceinte. De plus, sa prescription très réglementée (limitée aux médecins hospitaliers) limite son emploi.

Les toxidermies prurigineuses sont actuellement traitées au moyen de corticoïdes locaux et/ou d'anti-histaminiques : leur traitement présente donc les mêmes inconvénients que ceux indiqués ci-dessus.

Les prurits sévères sont aussi traités avec des corticoïdes locaux avec les mêmes inconvénients que ceux indiqués ci-dessus.

Il subsiste donc le besoin d'un traitement de ces affections de la peau, ne présentant pas ces inconvénients.

La présente invention a justement pour objet l'utilisation, dans un milieu cosmétiquement, pharmaceutiquement et/ou dermatologiquement acceptable, d'un ou plusieurs antagonistes de CGRP permettant de traiter efficacement certaines maladies de peau, tout en remédiant aux inconvénients mentionnés ci-dessus.

Le CGRP est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

Cependant personne n'avait jusqu'à ce jour envisagé de traiter les lichens, les prurigos, les toxidermies prurigineuses et les prurits sévères au moyen d'antagonistes de CGRP.

La présente invention a donc pour objet l'utilisation d'au moins un antagoniste de CGRP dans et/ou pour la préparation d'une composition cosmétique, pharmaceutique et/ou dermatologique pour traiter les lichens plans, les prurigos, choisis parmi les prurigos actiniques, de Besnier, ou Hebra, ou Strophulus et les toxidermies prurigineuses.

La demanderesse définit un antagoniste de CGRP comme toute molécule d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment aux caractéristiques suivantes :
- avoir une affinité pour les récepteurs au CGRP et/ou
- avoir une activité pharmacologique antagoniste du CGRP, c'est-à-dire induire une réponse pharmacologique cohérente notamment dans l'un des tests suivants :
   - la substance antagoniste doit diminuer la vasodilation induite par la capsaïcine et/ou
   - la substance antagoniste doit provoquer une inhibition de la libération de CGRP par les fibres nerveuses sensitives et/ou
   - la substance antagoniste doit diminuer une inhibition de la contraction du muscle lisse du canal déférent induite par le CGRP.

On peut utiliser dans l'invention par exemple comme antagoniste de CGRP, le CGRP 8-37, un anticorps anti-CGRP.

Dans les compositions selon l'invention, l'antagoniste de CGRP est utilisé de préférence en une quantité allant de 0,000001 à 10 % en poids par rapport au poids total de la composition, et en particulier en une quantité allant de 0,0001 à 5 % en poids par rapport au poids total de la composition.

De façon avantageuse, on peut associer à l'antagoniste de CGRP un ou plusieurs antagonistes d'un autre neuropeptide comme des antagonistes de substance P, et/ou un ou plusieurs antagonistes de médiateur de l'inflammation comme des antagonistes d'histamine, des antagonistes d'interleukine 1 (IL1), et des antagonistes de Tumor Necrosis Factor alpha (TNF α).

Aussi, l'invention a encore pour objet une composition pharmaceutique et/ou dermatologique, caractérisée en ce qu'elle contient, dans un milieu pharmaceutiquement et/ou dermatologiquement acceptable, au moins un antagoniste de CGRP et au moins un antagoniste d'un autre neuropeptide et/ou au moins un antagoniste d'un médiateur de l'inflammation.

L'invention a aussi pour objet une composition pharmaceutique et/ou dermatologique pour le traitement des lichens plans, caractérisée en ce qu'elle contient, dans un milieu pharmaceutiquement et/ou dermatologiquement acceptable, au moins un antagoniste de CGRP et au moins un antagoniste d'un autre neuropeptide et/ou au moins un antagoniste d'un médiateur de l'inflammation.

L'antagoniste de neuropeptide autre que le CGRP est de préférence un antagoniste de substance P.

La substance P est un élément chimique polypeptidique élaboré et libéré par une terminaison nerveuse. Elle fait partie de la famille des tachykinines qui proviennent des terminaisons nerveuses libres de l'épiderme et du derme. La substance P intervient notamment dans la transmission de la douleur et dans des maladies du système nerveux central tels que l'anxiété, la schizophrénie, dans des maladies respiratoires et inflammatoires, dans des maladies gastro-intestinales, dans des maladies rhumatismales et dans certaines maladies dermatologiques telles que l'eczéma, le psoriasis, l'urticaire et les dermites de contact.

Comme antagoniste de substance P utilisable dans l'invention, on peut citer toute substance d'origine organique ou minérale, capable de produire une inhibition de la fixation réceptorielle de substance P ou une inhibition de la synthèse et/ou la libération de substance P par des fibres nerveuses sensitives.

Pour qu'une substance soit reconnue comme un antagoniste de substance P, elle doit répondre aux caractéristiques suivantes :
- avoir une affinité sélective pour les récepteurs NK1 des tachykinines et/ou
- avoir une activité pharmacologique antagoniste de la substance P, c'est-à-dire induire une réponse pharmacologique cohérente notamment dans l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P est de préférence un antagoniste réceptoriel de substance P.

L'antagoniste réceptoriel de substance P peut être un peptide ou un dérivé non peptidique comportant un hétéroatome, et plus précisément un composé comportant un hétérocycle ou un hétéroatome lié directement ou indirectement à un cycle benzènique.

On peut utiliser par exemple comme peptide antagoniste réceptoriel de substance P le sendide et le spantide II.

On peut également utiliser dans l'invention comme peptide ceux décrits dans les documents US-A-4472305, US-A4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes réceptoriels de substance P non peptidiques utilisables dans l'invention sont notamment des composés hétérocycliques notamment soufrés, azotés ou oxygénés ou des composés comprenant un atome d'azote lié directement ou indirectement à un cycle benzénique.

Comme composé hétérocyclique, on peut utiliser dans l'invention ceux décrits dans les documents suivants : EP-A-360390, EP-A429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme composés comportant un atome d'azote lié directement ou indirectement à un noyau benzénique, on peut citer ceux décrits dans les documents suivants : EP-A-522808, WO-A-93/01165.

Comme antagonistes de médiateur de l'inflammation utilisables dans l'invention, on peut citer les dérivés de la diéthylène diamine telle que cinnarizine, cyclizine ; les dérivés de l'aminopropane (dexchlorophéniramine, tripolidine) ; des dérivés de phénothiazine (alimémazine, prométhazine) ; l'auranofine ; la lisophyline ; l'A802715 ; la sulfasalazine ; la cétirizine HCl ; la loratidine ; l'esbatine ; la sétastine HCI.

A titre d'exemple, les antagonistes de substance P et les antagonistes de médiateur de l'inflammation peuvent être utilisés en une quantité représentant de 0,000001 à 10 % du poids total de la composition et mieux de 0,0001 à 5 %.

La composition de l'invention peut être appliquée soit par voie locale, c'est-à-dire par voie topique ou par injection sous-cutanée et/ou intradermique, soit par voie systémique ou générale, c'est-à-dire par voie orale et/ou injection intramusculaire.

La composition de l'invention destinée à l'application topique contient un milieu pharmaceutiquement ou dermatologiquement acceptable, c'est-à-dire un milieu compatible avec la peau, les ongles, les muqueuses, les tissus et les cheveux. La composition contenant l'antagoniste de CGRP peut être appliquée par voie topique sur le visage, le cou, les cheveux, les muqueuses et les ongles, les grands plis ou toute autre zone cutanée du corps.

Les compositions selon l'invention peuvent se présenter sous toutes les formes galéniques normalement utilisées selon la voie d'utilisation (injection, orale, topique).

Pour une application topique, la composition peut se présenter notamment sous toutes les formes galéniques normalement utilisées pour une telle application, notamment sous forme de solutions aqueuses, hydroalcooliques ou huileuses, de dispersions du type lotion ou sérum, de gels aqueux, anhydres ou lipophiles, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les compositions injectables peuvent se présenter sous forme d'une lotion aqueuse, huileuse ou sous forme de sérum.

Les compositions utilisées par voie orale peuvent se présenter sous forme de capsules, de gélules, de sirops ou de comprimés.

Les quantités des différents constituants des compositions selon l'invention sont celles classiquement utilisées dans les domaines considérés.

Ces compositions constituent notamment des crèmes de protection, de traitement ou de soin pour le visage, pour les mains, pour les pieds, pour les grands plis anatomiques ou pour le corps, des laits corporels de protection ou de soin, des lotions, gels ou mousses pour le soin de la peau et des muqueuses, comme des lotions de nettoyage ou de désinfection, des compositions pour le bain, des compositions contenant un agent bactéricide.

Les compositions peuvent également consister en des préparations solides constituant des savons ou des pains de nettoyage.

Les quantités des différents constituants des compositions selon l'invention sont celles classiquement utilisées dans les domaines considérés.

L'invention peut être mise en oeuvre notamment en appliquant les compositions telles que définies ci-dessus, selon la technique d'utilisation habituelle de ces compositions. Par exemple : application de crèmes, de gels, de sérums, de lotions, de laits sur la peau, le cuir chevelu et/ou les muqueuses.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition pharmaceutique ou dermatologique de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le PPG-3 Myristyl éther et la cétyl diméthicone copolyol.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les antagonistes de CGRP à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc;
- les antiacnéiques comme l'acide rétinoïque ou le peroxyde de benzoyle.

Les compositions suivantes illustrent l'invention : les proportions indiquées sont des pourcentages en poids.

### Exemple 1 : Lotion désinfectante pour le visage ou les muqueuses

| | |
|---|---|
| CGRP 8-37 | 0,50 |
| Antioxydant | 0,05 |
| Isopropanol | 40,00 |
| Conservateur | 0,30 |
| Eau | qsp 100% |

Cette composition peut être utilisée aussi bien pour traiter les lichens plans ou pigmentaires ainsi que les toxidermies prurigineuses.

### Exemple 2 : Gel pour le visage ou le corps pour le traitement du lichen plan

| | |
|---|---|
| Hydroxypropylcellulose (Klucel H vendu par la société Hercules) | 1,00 |
| CGRP 8-37 | 0,0001 |
| Acide salicylique | 1,00 |
| Antioxydant | 0,05 |
| Isopropanol | 40,00 |
| Conservateur | 0,30 |
| Eau | qsp 100% |

### Exemple 3 : Crème (émulsion huile-dans-eau) pour le traitement des prurits sévères

| | |
|---|---|
| Anticorps Anti-CGRP | 0,05 |
| Stéarate de glycérol | 2,00 |
| Acide lactique/acide acétique | 1,00 |
| Polysorbate 60 (Tween 60 vendu par la société ICI) | 1,00 |
| Acide stéarique | 1,40 |
| Triéthanolamine | 0,70 |
| Carbomère | 0,40 |
| Huile siliconée | 12,00 |
| Perhydrosqualène | 12,00 |
| Antioxydant | 0,05 |
| Parfum | 0,5 |
| Conservateur | 0,30 |
| Eau | qsp 100% |

### Exemple 4 : Gel

L'exemple 4 est identique à l'exemple 3, à l'exception du fait qu'il contient en plus 0,3 % de sendide.

### Exemple 5 : Gel pour le traitement des lichens

| | |
|---|---|
| Sendide | 1,00 |
| Anticorps anti-CGRP | 0,10 |
| Hydroxypropylcellulose (Klucel H) | 1,00 |
| Antioxydant | 0,05 |
| Isopropanol | 40,00 |
| Conservateur | 0,30 |
| Eau | qsp 100% |

### Exemple 6 : Crème (émulsion huile-dans-eau) pour les prurits anales

| | |
|---|---|
| Anticorps anti-CGRP | 2,00 |
| Cétyldiméthicone copolyol | 2,50 |
| NaCl | 0,60 |
| NaOH | qsp pH = 5 |
| Cyclométhicone | 18,00 |
| PPG-3 myristyl éther | 6,00 |
| Glycérine | 3,00 |
| Conservateur | 0,20 |
| Eau | qsp 100% |

## Revendications

1. Utilisation d'au moins un antagoniste de CGRP pour la préparation d'une composition cosmétique, pharmaceutique et/ou dermatologique destinée à traiter les lichens plans, les prurigos choisis parmi les prurigos actiniques, de Besniers ou Hebra ou strophulus et les toxidermies prurigineuses.

2. Utilisation selon la revendication précédente, **caractérisée en ce que** l'antagoniste de CGRP est une molécule qui diminue la vasodilatation Induite par la capsaïcine.

3. Utilisation selon l'une quelconque des revendications précédentes, **caractérisée en ce que** l'antagoniste de CGRP est une molécule qui diminue une inhibition de la contraction du muscle lisse du canal déférent, induite par le CGRP.

4. Utilisation selon l'une quelconque des revendications précédentes, **caractérisée en ce que** l'antagoniste de CGRP est une molécule choisie parmi le CGRP 8-37 et les anticorps anti-CGRP.

5. Utilisation selon l'une quelconque des revendications 1 à 4, **caractérisée en ce que** l'antagoniste de CGRP est utilisé en une quantité allant de 0,000001 à 10 % en poids par rapport au poids total de la composition.

6. Utilisation selon l'une quelconque des revendications précédentes, **caractérisée en ce que** l'antagoniste de CGRP est utilisé en une quantité allant de 0,0001 à 5 % en poids par rapport au poids total de la composition.

7. Utilisation selon l'une quelconque des revendications précédentes, **caractérisée en ce que** la composition contient, en outre, au moins un agent choisi parmi les antagonistes de neuropeptide autre que le CGRP, les antagonistes de médiateur de l'inflammation, les agents antibactériens, antiparasitaires, antifongiques, anti-inflammatoires, antiprurigineux, anesthé-siques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

8. Utilisation selon l'une quelconque des revendications précédentes, **caractérisée en ce que** la composition contient, en outre, au moins un antagoniste de substance P, un antagoniste d'histamine, un antagoniste d'interleukine 1 et/ou un antagoniste de TNFα.

9. Utilisation selon la revendication 8, **caractérisée en ce que** l'antagoniste est utilisé en une quantité représentant de 0,0001 à 5 % du poids total de la composition.

10. Utilisation selon l'une quelconque des revendications précédentes, **caractérisée en ce que** la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles, les hydroxyacides.

11. Utilisation selon l'une quelconque des revendications précédentes, **caractérisée en ce que** la composition est une solution aqueuse, huileuse ou hydroalcoolique, une émulsion eau-dans-huile, une émulsion hulle-dans-eau, une microémulsion, un gel aqueux, un gel anhydre, un sérum, une dispersion de vésicules, de microcapsules ou de microparticules.

12. Utilisation selon l'une quelconque des revendications précédentes, **caractérisée en ce que** la composition contient également des adjuvants tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.

13. Composition pharmaceutique et/ou dermatologique à application topique, destinée à traiter les lichens plans, les prurigos choisis parmi les prurigo aotiniques, de Besmer ou Hebra ou Strophilus, et les toxidermies prurigineuses **caractérisée en ce qu'**elle contient, dans un milieu pharmaceutiquement et/ou dermatologiquement acceptable, au moins un antagoniste de CGRP et au moins un antagoniste d'un autre neuropeptide et/ou au moins un antagoniste d'un médiateur de l'inflammation.

14. Composition selon la revendication 13, **caractérisée en ce que** l'antagoniste de CGRP est une molécule qui diminue la vasodilatation indulte par la capsaïcine.

15. Composition selon la revendication 13 ou 14, **caractérisée en ce que** l'antagoniste de CGRP est une molécule qui diminue une inhibition de ta contraction du muscle lisse du canal déférent, induite par le CGRP.

16. Composition selon l'une quelconque des revendications 13 à 15, **caractérisée en ce que** l'antagoniste de CGRP est une molécule choisie parmi le CGRP 8-37 et les anticorps anti-CGRP.

17. Composition selon l'une quelconque des revendications 13 à 16, **caractérisée en ce que** l'antagoniste de CGRP est utilisé en une quantité allant de 0.00001 à 10% en poids par rapport au poids total de la composition.

18. Composition salon l'une quelconque des revendications 13 à 17, **caractérisée en ce que** l'antagoniste de CGRP est utilisé en une quantité allant de 0,0001 à 5% en poids par rapport au poids total de la composition.

19. Composition selon l'une quelconque des revendications 13 à 18, **caractérisée en ce que** l'antagoniste du neuropeptide autre que le CGRP est choisi parmi les antagonistes de substance P.

20. Composition selon la revendication 19, **caractérisée en ce que** l'antagoniste de substance P est un antagoniste réceptoriel de substance P.

21. Composition selon l'une quelconque des revendications 13 à 20, **caractérisée en ce que** l'antagoniste de médiateur de l'inflammation est choisi parmi les antagonistes d'histamine, les antagonistes d'interleukine 1 et les antagonistes de TNFα.

22. Composition selon l'une quelconque des revendications 13 à 21, **caractérisée en ce que** l'antagoniste de substance P ou l'antagoniste de médiateur de l'inflammation est utilisé en une quantité représentant de 0,0001 à 5 % du poids total de la composition.

23. Composition selon rune quelconque des revendications 13 à 22. **caractérisée en ce qu'**elle contient en outre au moins un agent choisi parmi les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée.

24. Composition selon l'une quelconque des revendications 13 à 23, **caractérisée en ce que** la milieu pharmaceutiquement et/ou dermatologiquement acceptable est une solution aqueuse, huileuse ou hydroalcoolique, une émulsion eau-dans-huile, une émulsion huile-dans-eau, une mir-roémulsion, un gel aqueux, un gel anhydre, un sérum, une dispersion de vésicules.

25. Composition selon l'une quelconque des revendications 13 à 24, **caractérisée en ce que** la composition contient également des adjuvants tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.

## Claims

1. Use of at least one CGRP antagonist in the preparation of a cosmetic, pharmaceutical and/or dermatological composition for the treatment of lichen planus, prurigos chosen from actinic prurigo, Besnier's or Hebra's prurigo or strophulus, and pruriginous toxicoderma.

2. Use according to the preceding claim, **characterized in that** the CGRP antagonist is a molecule which decreases capsaicin-induced vasodilation.

3. Use according to any one of the preceding claims, **characterized in that** the CGRP antagonist is a molecule which decreases a CGRP-induced inhibition of vas deferens smooth muscle contraction.

4. Use according to any one of the preceding claims, **characterized in that** the CGRP antagonist is a molecule chosen from CGRP 8-37 and anti-CGRP antibodies.

5. Use according to any one of Claims 1 to 4, **characterized in that** the CGRP antagonist is used in an amount ranging from 0.000001 to 10% by weight with respect to the total weight of the composition.

6. Use according to any one of the preceding claims, **characterized in that** the CGRP antagonist is used in an amount ranging from 0.0001 to 5% by weight with respect to the total weight of the composition.

7. Use according to any one of the preceding claims, **characterized in that** the composition also contains at least one agent chosen from antagonists of neuropeptides other than CGRP, inflammation mediator antagonists, antibacterial agents, antiparasitic agents, antifungal agents, anti-inflammatory agents, anti-pruriginous agents, anaesthetics, antiviral agents, keratolytic agents, anti-free-radical agents, antiseborrhoeic agents, antidandruff agents, anti-acne agents and/or agents which modify the differentiation and/or proliferation and/or pigmentation of the skin.

8. Use according to any one of the preceding claims, **characterized in that** the composition also contains at least one substance P antagonist, one histamine antagonist, one interleukin 1 antagonist and/or one TNFα antagonist.

9. Use according to Claim 8, **characterized in that** the antagonist is used in an amount representing from 0.0001 to 5% of the total weight of the composition.

10. Use according to any one of the preceding claims, **characterized in that** the composition also contains at least one active agent chosen from proteins or protein hydrolysates, amino acids, polyols, urea, sugars and sugar derivatives, vitamins, starch, plant extracts, essential fatty acids, ceramides, essential oils and hydroxy acids.

11. Use according to any one of the preceding claims, **characterized in that** the composition is an aqueous, oily or aqueous-alcoholic solution, a water-in-oil emulsion, an oil-in-water emulsion, a microemulsion, an aqueous gel, an anhydrous gel, a serum, or a dispersion of vesicles, of microcapsules or of microparticles.

12. Use according to any one of the preceding claims, **characterized in that** the composition also contains adjuvants such as hydrophilic or lipophilic gelling agents, hydrophilic or lipophilic active agents; preserving agents, antioxidants, solvents, fragrances, fillers, screening agents, bactericides, odour absorbers and dyestuffs.

13. Pharmaceutical and/or dermatological composition for topical application for the treatment of lichen planus, prurigos chosen from actinic prurigo, Besnier's or Hebra's prurigo or strophulus, and pruriginous toxicoderma **characterized in that** it contains, in a pharmaceutically and/or dermatologically acceptable medium, at least one CGRP antagonist and at least one antagonist of another neuropeptide and/or at least one inflammation mediator antagonist.

14. Composition according to Claim 13, **characterized in that** the CGRP antagonist is a molecule which decreases capsaicin-induced vasodilation.

15. Composition according to Claim 13 or 14, **characterized in that** the CGRP antagonist is a molecule which decreases a CGRP-induced inhibition of vas deferens smooth muscle contraction.

16. Composition according to any one of Claims 13 to 15, **characterized in that** the CGRP antagonist is a molecule chosen from CGRP 8-37 and anti-CGRP antibodies.

17. Composition according to any one of Claims 13 to 16, **characterized in that** the CGRP antagonist is used in an amount ranging from 0.00001 to 10% by weight with respect to the total weight of the composition.

18. Composition according to any one of Claims 13 to 17, **characterized in that** the CGRP antagonist is used in an amount ranging from 0.0001 to 5% by weight with respect to the total weight of the composition.

19. Composition according to any one of Claims 13 to 18, **characterized in that** the antagonist of the neuropeptide other than CGRP is chosen from substance P antagonists.

20. Composition according to Claim 19, **characterized in that** the substance P antagonist is a substance P receptor antagonist.

21. Composition according to any one of Claims 13 to 20, **characterized in that** the inflammation mediator antagonist is chosen from histamine antagonists, interleukin 1 antagonists and TNFα antagonists.

22. Composition according to any one of Claims 13 to 21, **characterized in that** the substance P antagonist or the inflammation mediator antagonist is used in an amount representing from 0.0001 to 5% of the total weight of the composition.

23. Composition according to any one of Claims 13 to 22, **characterized in that** it also contains at least one agent chosen from antibacterial agents, antiparasitic agents, antifungal agents, anti-inflammatory agents, antiseptics, anti-pruriginous agents, anaesthetics, antiviral agents, keratolytic agents, anti-free-radical agents, antiseborrhoeic agents, antidandruff agents, anti-acne agents and agents which modify the differentiation and/or proliferation and/or pigmentation of the skin.

24. Composition according to any one of Claims 13 to 23, **characterized in that** the pharmaceutically and/or dermatologically acceptable medium is an aqueous, oily or aqueous-alcoholic solution, a water-in-oil emulsion, an oil-in-water emulsion, a microemulsion, an aqueous gel, an anhydrous gel, a serum or a dispersion of vesicles.

25. Composition according to any one of Claims 13 to 24, **characterized in that** the composition also contains adjuvants such as hydrophilic or lipophilic gelling agents, hydrophilic or lipophilic active agents, preserving agents, antioxidants, solvents, fragrances, fillers, screening agents, bactericides, odour absorbers and dyestuffs.

## Patentansprüche

1. Verwendung mindesten seines CGRP-Antagonisten für die Herstellung einer kosmetischen, pharmazeutischen und/oder dermatologischen Zusammensetzung zur Behandlung von Lichen planus, Prurigo, die unter aktinischer Prurigo, Prurigo Besnier, Prurigo Hebra oder Strophulus ausgewählt ist, und pruriginösen Toxidermien.

2. Verwendung nach dem vorhergehenden Anspruch, **dadurch gekennzeichnet, dass** der CGRP-Antagonist ein Molekül ist, das die durch Capsaicin hervorgerufene Vasodilatation verringert.

3. Verwendung nach einem der vorhergehenden Ansprüche, **dadurch gekennzeichnet, dass** der CGRP-Antagonist ein Molekül ist, das eine Inhibierung der Kontraktion der glatten Muskulatur des Samenleiters, die durch CGRP hervorgerufen wird, vermindert,

4. Verwendung nach einem der vorhergehenden Ansprüche, **dadurch gekennzeichnet, dass** der CGRP-Antagonist unter CGRP 8-37 und anti-CGRP-Antikörpern ausgewählt ist.

5. Verwendung nach einem der Ansprüche 1 bis 4, **dadurch gekennzeichnet, dass** der CGRP-Antagonist in einem Mengenanteil von 0,000001 bis 10 Gew.-%, bezogen auf das Gesamtgewicht der Zusammensetzung, verwendet wird.

6. Verwendung nach einem der vorhergehenden Ansprüche, **dadurch gekennzeichnet, dass** der CGRP-Antagonist in einem Mengenanteil von 0,0001 bis 5 Gew.-%, bezogen auf das Gesamtgewicht der Zusammensetzung, verwendet wird.

7. Verwendung nach einem der vorhergehenden Ansprüche, **dadurch gekennzeichnet, dass** die Zusammensetzung ferner mindestens eine Substanz enthält, die unter den von den CGRP-Antagonisten verschiedenen Neuropeptid-Antagonisten, Antagonisten von Entzündungsmediatoren, antibakteriellen Wirkstoff fen, antiparasitären Wirkstoffen, Wirkstoffen gegen Filze, entzündungshemmenden Wirkstoffen, Wirkstoffen gegen Juckreiz, Anästhetika, Wirkstoffen gegen Viren, Kex-atolytika, Radikalfängern für freie Radikale, Wirkstoffen gegen Seborrhoe, Wirkstoffen gegen Schuppen, Wirkstoffen gegen Akne und/oder Wirkstoffen, die die Differenzierung und/oder Proliferation und/oder Pigmentierung der Haut modulieren, ausgewählt ist.

8. Verwendung nach einem der vorhergehenden Ansprüche, **dadurch gekennzeichnet, dass** die Zusammensetzung ferner mindestens einen Substanz P-Antagonisten, Histamin-Antagonisten, Interleukin 1-Antagonisten und/oder TNF-α-Antagonisten enthält.

9. Verwendung nach Anspruch 8, **dadurch gekennzeichnet, dass** der Antagonist in einem Mengenanteil von 0,0001 bis 5 % des Gesamtgewichts der Zusammensetzung verwendet wird.

10. Verwendung nach einem der vorhergehenden Ansprüche, **dadurch gekennzeichnet, dass** die Zusammensetzung ferner mindestens einen Wirkstoff enthält, der unter den Proteinen oder Proteinhydrolysaten, Aminosäuren, Polyolen, Harnstoff, Zuckern und Zuckerderivaten, Vitaminen, Stärke, Pflanzenextrakten, essentiellen Fettsäuren, Ceramiden, etherischen Ölen und Hydroxysäuren ausgewählt ist.

11. Verwendung nach einem der vorhergehenden Ansprüche, **dadurch gekennzeichnet, dass** die Zusammensetzung eine wässrige, ölige oder wässrig-alkoholische Lösung, eine Wasser-in-Öl-Emulsion, eine Öl-in-Wasser-Emulsion, eine Mikroemulsion, ein wässriges Gel, ein wasserfreies Gel, ein Serum, eine Vesikeldispersion, eine Mikrokapseldispersion oder eine Mikropartikeldispersion ist.

12. Verwendung nach einem der vorhergehenden Ansprüche, **dadurch gekennzeichnet, dass** die Zusammensetzung auch Zusatzstoffe enthält, wie lipophile oder hydrophile Gelbildner, lipophile oder hydrophile Wirkstoffe, Konservierungsmittel, Antioxidantien, Lösungsmittel, Parfums, Füllstoffe, Filter, Bakterizide, Geruchsabsorber und Färbemittel.

13. Pharmazeutische und/oder dermatologische Zusammensetzung, die für die Behandlung von Lichen planus, Prurigo, die unter aktinischer Prurigo, Prurigo Besnier, Prurigo Hebra oder Strophulus ausgewählt ist, und pruriginösen Toxidermien, **dadurch gekennzeichnet, dass** sie in einem pharmazeutisch und/oder dermatologisch akzeptablen Medium mindestens einen CGRP-Antagonisten und mindestens einen Antagonisten eines weiteren Neuropeptids und/oder einen Antagonisten eines Entzündungsmediators enthält.

14. Zusammensetzung nach Anspruch 13, **dadurch gekennzeichnet, dass** der CGRP-Antagonist ein Molekül ist, das die durch Capsaicin hervorgerufene Vasodilatation verringert.

15. Zusammensetzung nach Anspruch 13 oder 14, **dadurch gekennzeichnet, dass** der CGRP-Antagonist ein Molekül ist, das eine Inhibierung der Kontraktion der glatten Muskulatur des Samenleiters, die durch CGRP hervorgerufen wird, vermindert.

16. Zusammensetzung nach einem der Ansprüche 13 bis 15, **dadurch gekennzeichnet, dass** der CCxRP-Antagonist unter CGRP 8-37 und anti-CGRP-Antikörpern ausgewählt ist.

17. Zusammensetzung nach einem der Ansprüche 13 bis 16, **dadurch gekennzeichnet, dass** der CGRP-Antagonist in einem Mengenanteil von 0,000001 bis 10 Gew.-%, bezogen auf das Gesamtgewicht der Zusammensetzung, verwendet wird.

18. Zusammensetzung nach einem der Ansprüche 13 bis 17, **dadurch gekennzeichnet, dass** der CGRP-Antagonist in einem Mengenanteil von 0,0001 bis 5 Gew.-%, bezogen auf das Gesamtgewicht der Zusammensetzung, verwendet wird.

19. Zusammensetzung nach einem der Ansprüche 13 bis 18, **dadurch gekennzeichnet, dass** der von den CGRP-Antagonisten verschiedene Neuropeptid-Antagonist unter den Antagonisten der Substanz P ausgewählt ist.

20. Zusammensetzung nach Anspruch 19, **dadurch gekennzeichnet, dass** der Substanz P-Antagonist ein Rezeptor-Antagonist der Substanz P ist.

21. Zusammensetzung nach einem der Ansprüche 13 bis 20, **dadurch gekennzeichnet, dass** der Antagonist eines Entzündungsmediators unter den Histamin-Antagonisten, Interleukin 1-Antagonisten und/ oder TNF-α-Antagonisten ausgewählt ist.

22. Zusammensetzung nach einem der Ansprüche 13 bis 21, **dadurch gekennzeichnet, dass** der Substanz P-Antagonist oder der Antagonist eines Entzündungsmediators in einem Mengenanteil von 0,0001 bis 5 % des Gesamtgewichts der Zusammensetzung verwendet wird.

23. Zusammensetzung nach einem der Ansprüche 13 bis 22, **dadurch gekennzeichnet, dass** sie ferner mindestens eine Substanz enthält, die unter den antibakteriellen Wirkstoffen, antiparasitären Wirkstoffen, Wirkstoffen gegen Pilze, entzündungshemmenden Wirkstoffen, antiseptischen Wirkstoffen, Wirkstoffen gegen Juckreiz, Anästhetika, Wirkstoffen gegen Viren, Keratolytika, Radikalfängern für freie Radikale, Wirkstoffen gegen Seborrhoe, Wirkstoffen gegen Schuppen, Wirkstoffen gegen Akne und/oder Wirkstoffen, die die Differenzierung und/oder Proliferation und/oder Pigmentierung der Haut modulieren, ausgewählt ist.

24. Zusammensetzung nach einem der Ansprüche 13 bis 23, **dadurch gekennzeichnet, dass** das pharmazeutisch und/ oder dermatologisch akzeptable Medium eine wässrige, ölige oder wässrig-alkoholische Lösung, eine Wasser-in-Öl-Emulsion, eine Öl-in-Wasser-Emulsion, eine Mikroemulsion, ein wässriges Gel, ein wasserfreies Gel, ein Serum oder eine Vesikeldispersion ist.

25. Zusammensetzung nach einem der Ansprüche 13 bis 24, **dadurch gekennzeichnet, dass** die Zusammensetzung auch Zusatzstoffe enthält, wie lipophile oder hydrophile Gelbildner, lipophile oder hydrophile Wirkstoffe, Konservierungsmittel, Antioxidantien, Lösungsmittel, Parfüms, Füllstoffe, Filter, Bakterizide, Geruchsabsorber und Färbemittel.
